# EUROPÄISCHE PATENTSCHRIFT

(11) **EP 0 499 925 B1**
(45) Veröffentlichungstag und Bekanntmachung des Hinweises auf die Patenterteilung: **18.10.1995**
(21) Anmeldenummer: 92102155.6
(22) Anmeldetag: 10.02.1992
(51) Int. Cl.: C07C 15/16, C07C 17/08

(54) **Verfahren zur Herstellung von 2,2-Bis-(3,4-dimethyl-phenyl)-propan**
Process for the preparation of 2,2-Bis-(3,4-Dimethyl-Phenyl)-propane
Procédé pour la préparation du 2,2-bis-(3,4-diméthyl-phényl)-propane

(30) Priorität: 22.02.1991 DE 4105532; 27.09.1991 DE 4132211
(43) Veröffentlichungstag der Anmeldung: 26.08.1992
(60) Teilanmeldung: 95105012.9
(73) Patentinhaber: BAYER AG, 51368 Leverkusen (DE)
(72) Erfinder: Arlt, Dieter, Prof. Dr., W-5000 Köln 80 (DE)

(56) Entgegenhaltungen:
- EP-A- 0 399 284
- US-A- 2 712 543
- THE JOURNAL OF ORGANIC CHEMISTRY. Bd. 4, Nr. 4, September 1939, BALTIMORE US. Seiten 428 - 435; M. S. KHARASCH ET AL.: 'the peroxide effect in the addition of reagents to unsaturated compounds. XXI. ...'
- HELVETICA CHIMICA ACTA Bd. 10, 15. März 1927, BASEL CH Seiten 132 - 139; HENRY GOUDET ET AL.: 'recherches sur quelques dérivés du propylène'

## Beschreibung

Die Erfindung betrifft ein neues Verfahren zur Herstellung von 2,2-Bis-(3,4-dimethyl-phenyl)-propan.

1,1-Diarylalkane sind im allgemeinen leicht durch säurekatalysierte Alkylierung von aromatischen Verbindungen mit Aldehyden oder Styrolen erhältlich (siehe z.B. J. Org. Chem. 26 (1961), 1398). Die Herstellung von geminalen Diarylalkanen mit substituierten Phenylgruppen, die im Prinzip durch Kondensation von aliphatischen Ketonen oder α-Methylstyrolen mit aromatischen Verbindungen erfolgen könnte, ist jedoch auf die Verwendung aktivierter aromatischer Verbindungen wie Phenole und Aniline beschränkt. Toluol z.B. läßt sich nicht mit Aceton oder α-Methylstyrolen zu den entsprechenden 2,2-Diarylpropanen umsetzen (siehe J. Org. Chem. loc. cit).

Die Herstellung geminaler Diarylalkane mit substituierten Phenylgruppen, z.B. des 2,2-Ditolylpropans, bereitet erhebliche Schwierigkeiten und erfordert für jede aromatische Verbindung jeweils spezielle Alkylierungsmittel und Friedel-Crafts-Katalysatoren (siehe J. Org. Chem. loc. cit. und J. Org. Chem. 41 (1976), 1698 ff).

In neuerer Zeit (DE-OS 3 916 557) wurde ein allgemein anwendbares Verfahren zur Herstellung von 2,2-Diaryl-alkanen, insbesondere von 2,2-Diaryl-propanen bekannt, das auf der Umsetzung von bestimmten Tetrachlorbutanen bzw. Trichlorbutenen mit aromatischen Kohlenwasserstoffen basiert. Dieses Verfahren setzt voraus, daß als Ausgangsstoff Tetrachlorkohlenstoff eingesetzt wird, so daß bei der Durchführung des Verfahrens ein erheblicher Aufwand zum Schutz der Umwelt entsteht. Außerdem fällt bei diesem Verfahren als Koppelprodukt 1,1-Dichlorethen an, das zwar als Monomer für spezielle Kunststoffe genutzt werden kann, dessen Menge aber nicht dem jeweiligen Bedarf angepaßt werden kann.

Da 2,2-Bis-(3,4-dimethyl-phenyl)-propan ein geeignetes Vorprodukt für die durch Oxidation zugängliche entsprechende Tetracarbonsäure ist, die ihrerseits wieder eine interessante Ausgangsverbindung für die Herstellung hochwertiger Kunststoffe (z.B. Polyimide) darstellt, bestand ein Bedarf an einem wirtschaftlichen und ökologisch unbedenklichen Verfahren zur Herstellung von 2,2-Bis-(3,4-dimethyl-phenyl)-propan.

Die Alkylierung von aromatischen Kohlenwasserstoffen mit bifunktionellen Alkylierungsmitteln wie 2,2-Dichlorpropan oder 2-Chlorpropen bzw. 2-Brom-propen oder mit reaktiven Zwischenstufen, die aus 2-Chlor-2-aryl-propanen gebildet werden und die vermutlich auch intermediär bei der stufenweise verlaufenden Alkylierung ausgehend von den genannten bifunktionellen Alkylierungsmitteln auftreten, ist ein sehr komplexer Vorgang, der zu einer Vielzahl von Reaktionsprodukten führt und häufig nur unbefriedigende Ausbeuten an erwünschten 2,2-Diaryl-propanen ergibt.

Aus der US-Patentschrift 2 712 543 ist es bekannt, 2,2-Bis-(3,4-dimethyl-phenyl)-propan aus o-Xylol und 2,2-Dichlorpropan herzustellen. Das Molverhältnis von eingesetztem o-Xylol zu eingesetztem 2,2-Dichlorpropan betrug 4:1; die erhaltene Ausbeute schwankte zwischen 50 und 58 % der Theorie.

Überraschenderweise wurde gefunden, daß die Umsetzung von o-Xylol und 2,2-Dichlorpropan zu einer stark erhöhten Ausbeute an 2,2-Bis-(3,4-dimethyl-phenyl)-propan führt, wenn man das Molverhältnis Xylol/Dichlorpropan auf mindestens 5:1 anhebt.

Gegenstand der Erfindung ist daher ein Verfahren zur Herstellung von 2,2-Bis-(3,4-dimethyl-phenyl)propan 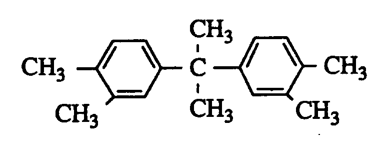
durch Umsetzung von o-Xylol mit 2,2-Dichlorpropan in Gegenwart von einem Friedel-Crafts-Katalysator, wobei das Molverhältnis von eingesetztem o-Xylol zu eingesetztem 2,2-Dichlorpropan mindestens 5:1, vorzugsweise mindestens 8:1, besonders bevorzugt mindestens 10:1, beträgt.

Die Art der für Friedel-Crafts-Alkylierungen geeigneten Katalysatoren ebenso wie die Reaktionsbedingungen sind im Prinzip bekannt; vgl. z.B. "Methoden der Organischen Chemie" (Houben-Weyl), Bd. V/1a, Georg Thieme Verlag, Stuttgart 1970, 501-539.

Das erfindungsgemäße Verfahren zur Herstellung von 2,2-Bis-(3,4-dimethyl-phenyl)-propan aus o-Xylol und 2,2-Dichlor-propan kann durch das folgende Schema 1 beschrieben werden. 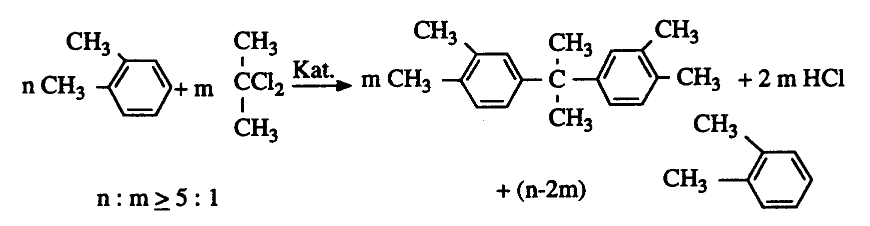
Das erfindungsgemäße Verfahren kann sowohl in der Flüssigphase als auch in der Gasphase durchgeführt werden. Bei der Durchführung des Verfahrens in der Flüssigphase wird im allgemeinen bei Temperaturen von -50 bis +200°C, vorzugsweise von -20 bis 180°C, besonders bevorzugt bei -10 bis +50°C gearbeitet. Als Friedel-Crafts-Katalysatoren können die üblicherweise für die Alkylierung von aromatischen Verbindungen verwendeten Friedel-Crafts-Katalysatoren eingesetzt werden, z.B. AlCl₃, BF₃, FeCl₃, ZnCl₂, TiCl₄, ZrCl₄, SnCl₄, H₃PO₄, HF, HBF₄, saure Erdalkaliphosphate, Tonerden, Zeolithe oder saure Ionenaustauscherharze. Gegebenenfalls kann zur Beschleunigung der Reaktion die Mitverwendung von Cokatalysatoren, wie sie bei der Friedel-Crafts-Alkylierung vielfach verwendet werden, vorteilhaft sein; solch ein Cokatalysator ist z.B. HCl.

Die Flüssigphasenalkylierung kann z.B. wie folgt ausgeführt werden: Die Lösung des Friedel-Crafts-Katalysators in überschüssigem o-Xylol wird unter Rühren bei Temperaturen von -10 bis +50°C mit 2,2-Dichlor-propan oder einer Lösung von 2,2-Dichlorpropan in o-Xylol versetzt. Das Reaktionsgemisch wird zur Vervollständigung der Umsetzung noch einige Zeit, je nach Ansatzgröße, Katalysator und gewählter Rekationstemperatur 10 Minuten bis 10 Stunden bei Temperaturen von 0 bis +50°C gerührt. Der Katalysator wird im allgemeinen anschließend durch Zugabe von Wasser desaktiviert. Die organische Phase des Reaktionsgemisches wird abgetrennt und nach dem Trocknen destillativ aufgearbeitet.

Bei Durchführung des erfindungsgemäßen Verfahrens in der Gasphase wird im allgemeinen bei Temperaturen von etwa 180 bis 450°C gearbeitet. Als Katalysatoren werden die üblicherweise für Gasphasenalkylierungen von Aromaten verwendeten Friedel-Crafts-Katalysatoren wie beispielsweise Al₂O₃.SiO₂, H₃PO₄.SiO₂, BF₃/γAl₂O₃ und saure Zeolithe angewendet.

Die Gasphasenalkylierung kann z.B. wie folgt ausgeführt werden: o-Xylol und 2,2-Dichlor-propan werden durch Erhitzen in einem geeigneten Apparat, z.B. einem Schlangenrohr- oder Fallfilm-Verdampfer, in den gasförmigen Zustand gebracht und in einen für kontinuierliche Gasphasen-Prozesse geeigneten Reaktor, z.B. einen Rohrbündel-Reaktor, überführt, der den Alkylierungskatslysator in fester Form enthält. Das den Reaktor verlassende Reaktionsgemisch wird durch Kondensation in flüssige und gasförmige Produkte aufgetrennt. Die Reaktion kann sowohl bei Normaldruck als auch unter vermindertem Druck ausgeführt werden.

Das 2,2-Bis-(3,4-dimethyl-phenyl)-propan und überschüssiges Xylol werden aus dem anfallenden Reaktionsgemisch durch fraktionierende Destillation erhalten bzw. zurückgewonnen, wobei Chlorwasserstoff durch Waschprozesse, z.B. mit Wasser, gegebenenfalls zuvor entfernt werden kann.

Zur weiteren Reinigung kann das erhaltene 2,2-Bis-(3,4-dimethyl-phenyl)-propan aus geeigneten organischen Lösungsmitteln umkristallisiert werden Geeignete organische Lösungsmittel umfassen z.B. Alkohole und aromatische oder aliphatische Kohlenwasserstoffe.

Eine besondere Ausführungsform des erfindungsgemäßen Verfahrens ist die Verwendung von 2-Chlor-propen bzw. von Gemischen von 2-Chlor-propen mit 1-Chlor-propenen, wie sie bei der Abspaltung von HCl aus 1,2-Dichlorpropan entstehen, als primärem Ausgangsstoff für die Alkylierung von o-Xylol zur Herstellung von 2,2-Bis-(3,4-dimethyl-phenyl)-propan.

Es ist bekannt, 2-Chlorpropen als Alkylierungsmittel für aromatische Kohlenwasserstoffe zur Herstellung von 2,2-Diaryl-propanen einzusetzen. Die Ausbeuten an erwünschtem Produkt sind häufig nicht zufriedenstellend. So ergibt die Umsetzung mit m-Xylol und 2-Chlorpropen (s. Helv. 10 (1927), S. 138) nur eine Ausbeute von 25 % der Theorie an 2,2-Bis-xylyl-propan.

Es wurde nun gefunden, daß man mit gutem Erfolg 2-Chlorpropen als Ausgangsstoff für die Herstellung von 2,2-Bis-(3,4-dimethyl-phenyl)-propan einsetzen kann, wenn man der Alkylierungsreaktion eine HCl-Addition in situ vorschaltet. Überraschenderweise stört die gleichzeitige Anwesenheit von 1-Chlorpropenen bei dieser Umsetzung von 2-Chlor-propen nicht.

Die Verwendung dieser Alkylierungsmittel - 2-Chlorpropen in reiner Form bzw. im Gemisch mit 1-Chlor-propenen, wobei das Gemisch vorzugsweise unter 80 Gew.-% 1-Chlorpropene (bezogen auf die Summe von 1- und 2-Chlorpropenen) enthält - ist vorteilhaft, weil dieser Ausgangsstoff leicht aus 1,2-Dichlorpropan durch HCl-Abspaltung zugänglich ist, wobei Gemische von 1- und 2-Chlor-propenen anfallen, deren aufwendige Trennung bei der vorgeschlagenen Verwendung entfallen kann. Das nach der Umsetzung anfallende, nicht umgesetzte 1-Chlor-propen kann von den anderen Ausgangs- und Reaktionsprodukten wegen der großen Siedepunktsunterschiede leicht abgetrennt werden.

Es wurde weiterhin gefunden, daß es möglich ist, durch selektive Umsetzung des 2-Chlorpropens, das im Gemisch mit 1-Chlorpropenen vorliegt, mit Chlorwasserstoff ein Gemisch aus 2,2-Dichlor-propan und 1-Chlorpropenen zu erhalten, das z.B. durch fraktionierende Destillation sehr leicht in 1-Chlorpropen-Gemisch und reines 2,2-Dichlorpropan aufgetrennt werden kann. Dadurch wird die aufwendige Trennung von 2- und 1-Chlorpropenen umgangen (2-Chlorpropen: Siedepunkt 22,6°C, E/Z-1-Chlorpropene: Siedebereich 30,8°C bis 37°C, 2,2-Dichlorpropan: Siedepunkt 69,5°C). Es ist bekannt, daß sowohl 2- als auch E- und Z-1-Chlorpropen bei der Umsetzung mit Chlorwasserstoff HCl-Additionsprodukte ergeben, aber es ist überraschend, daß die Addition an 2-Chlorpropen soviel schneller als an 1-Chlorpropene erfolgt, daß sich daraus ein vorteilhaftes Herstellungsverfahren für reines 2,2-Dichlorpropan ergibt. Die Umsetzung kann unter Normaldruck oder Überdruck durchgeführt werden; vorteilhaft wird im Bereich von 2 bis 50 bar gearbeitet. Die eingesetzte HCl-Menge sollte mindestens stöchiometrisch zur im Edukt-Gemisch enthaltenen 2-Chlorpropenmenge sein. Vorteilhaft werden die obengenannten Friedel-Crafts-Katalysatoren zur Beschleunigung der HCl-Addition eingesetzt.

Da 1,2-Dichlorpropan Zwangsanfall-Produkt bei der technischen Herstellung von Propenoxid ist, stellt die vorgeschlagene Verwendung von 2-Chlor-propen eine ökologisch besonders vorteilhafte Verfahrensweise zur Verfügung, da sie ein mit erheblichem Aufwand zu entsorgendes Abfallprodukt nutzbar macht.

Die Variante des erfindungsgemäßen Verfahrens zur Herstellung von 2,2-Bis-(3,4-dimethyl-phenyl)-propan, die von o-Xylol, 2-Chlorpropen und Chlorwasserstoff ausgeht, wird durch das Schema 2 beschrieben. 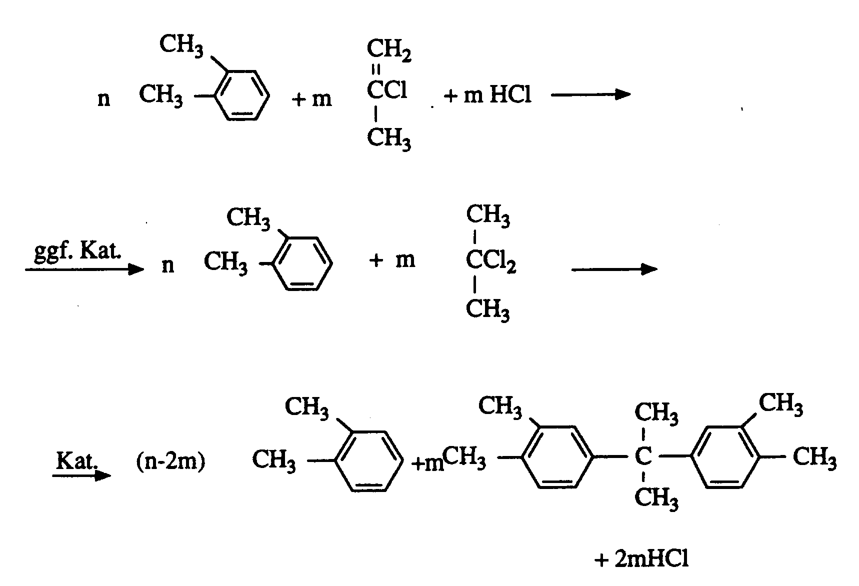
Dieses Verfahren wird im allgemeinen in der Flüssigphase bei Temperaturen von -78 bis +200°C, vorzugsweise bei -20 bis +180°C, besonders bevorzugt bei -10 bis +50°C, ausgeführt.

Die Umsetzung verläuft in zwei Stufen, die sowohl in getrennten Reaktoren als auch in einem Reaktor nacheinander ausgeführt werden können Bei einer diskontinuierlichen Arbeitsweise ist es zweckmäßig, die Reaktionen in dem gleichen Reaktor, bei kontiuierlicher Arbeitsweise ist es im allgemeinen zweckmäßig, die Reaktionen in getrennten Reaktoren durchzuführen.

In der ersten Reaktionsstufe wird gegebenenfalls in o-Xylol gelöstes 2-Chlorpropen bzw. ein Gemisch von 2-Chlor-propen und 1-Chlor-propenen mit Chlorwasserstoff umgesetzt, wobei 2,2-Dichlorpropan gebildet wird. Die Umsetzung kann in dieser ersten Reaktionsstufe unkatalysiert ausgeführt werden; zweckmäßig ist es jedoch, die HCl-Addition durch Zugabe geeigneter Katalysatoren zu beschleunigen. Geeignete Katalysatoren sind die üblicherweise zur Friedel-Crafts-Alkylierung angewendeten Katalysatoren, wie z.B. AlCl₃ oder HF.BF₃, wobei in dieser ersten Reaktionsstufe, sofern bereits o-Xylol als Lösungsmittel zur Anwendung kommt, vor allem mild wirkende Friedel-Crafts-Katalysatoren wie ZnCl₂, FeCl₃, BiCl₃, SnCl₄, TiCl₄, ZrCl₄, saure Tonerden, Zeolithe oder saure Ionenaustauscher, die bei den in der 1. Reaktionsstufe angewandten Temperaturen von -78 bis +90°C zwar die Addition von Chlorwasserstoff an 2-Chlorpropen, die unerwünschten Nebenreaktion von 2-Chlorpropen mit dem aromatischen Kohlenwasserstoff jedoch nicht merklich katalysieren, eingesetzt werden. Geeignet sind weiterhin Katalysatoren, die zwar die HCl-Addition an ungesättigte Verbindungen katalysieren, jedoch nicht als Friedel-Crafts-Katalysatoren bei der Alkylierung von aromatischen Kohlenwasserstoffen wirksam sind, wie z.B. Kupferhalogenide, Quecksilbersalze oder Bariumchlorid, wobei diese Stoffe auch in Form heterogener Katalysatoren auf Trägern, wie z.B. Aktivkohle, angewendet werden können.

Der gasförmige Chlorwasserstoff, der in der ersten Reaktionsstufe mit 2-Chlorpropen umgesetzt wird, wird in mindestens äquivalenter Menge eingesetzt, kann aber auch im Überschuß, z.B. 2 bis 10 Mol pro Mol 2-Chlorpropen, eingesetzt werden. Diese 1. Reaktionsstufe kann sowohl bei Normaldruck als auch unter Druck ausgeführt werden, wobei Druck zur Reaktionsbeschleunigung beiträgt. Der Druckbereich liegt zwischen 1 und 10 bar, im allgemeinen nicht über 8 bar.

Der Reaktionsablauf wird zweckmäßig durch gaschromatographische Untersuchung des Reaktionsgemisches verfolgt. Nach weitgehender Umsetzung des 2-Chlorpropens wird der zweite Reaktionsschritt ausgeführt.

Der zweite Reaktionsschritt ist die weitere Umsetzung des gebildeten 2,2-Dichlorpropans mit o-Xylol in der Flüssigphase, die oben bereits beschrieben wurde (Schema 1).

Sofern die erste Reaktionsstufe bereits in o-Xylol als Lösungsmittel unter Verwendung milder Friedel-Crafts-Katalysatren ausgeführt wurde, kann der zweite Reaktionsschritt, der mitunter parallel zur 2,2-Dichlorpropanbildung bereits langsam abläuft, durch Temperaturerhöhung beschleunigt werden. Zweckmäßig ist es jedoch, einen aktiven, stärker wirkenden Friedel-Crafts-Katalysator, der z.B. in o-Xylol gelöst bzw. suspendiert sein kann, dem Reaktionsgemisch hinzuzufügen. Auf jeden Fall müssen Friedel-Crafts-Katalysatoren hinzugefügt werden, wenn solche Katalysatoren in der ersten Reaktionsstufe noch nicht zur Anwendung kamen.

Die weiteren Verfahrensweise entspricht der bereits für die Umsetzung von 2,2-Dichlorpropan mit o-Xylol ausgeführten Beschreibung. Zur weiteren Aufarbeitung des Reaktionsgemisches werden zunächst gelöste oder suspendierte Katalysatoren durch wäßrige Wäsche oder Filtration entfernt. Dann kann eine fraktionierte Destillation durchgeführt werden. Dabei werden unumgesetzte Chlorpropene als Niedrigsieder abgetrennt und nach bekannten Verfahren, z.B. durch katalytische Hydrierung, entsorgt.

Eine bevorzugte Verfahrensweise nutzt die Kombination der intermediären Herstellung des Alkylierungsmittels 2,2-Dichlorpropan aus 2-Chlor-propen und Chlorwasserstoff und des im Alkylierungsschritt anfallenden Chlorwasserstoffs. Bei dieser Ausgestaltung des Verfahrens wird das anfallende Reaktionsgemisch zunächst destillativ aufgearbeitet, der anfallende Chlorwasserstoff wird bzw. die Chlorwasserstoff-haltigen Xylolfraktionen werden in die erste Reaktionsstufe zurückgeführt. Bei Verwendung eines heterogenen Festphasenkatalysators wird vor der destillativen Aufarbeitung dieser Katalysator durch Filtration entfernt. Bei Verwendung eines flüchtigen Katalysators, wie z.B. TiCl₄ oder HF.BF₃, wird dieser destillativ vom Reaktionsprodukt, gegebenenfalls zusammen mit o-Xylol als Mischfraktion, abgetrennt und der Reaktion erneut zugeführt. Bei Verwendung von nicht durch Filtration oder Destillation vom Reaktionsprodukt abgetrennbaren Katalysatoren, wie z.B. AlCl₃, wird der Katalysator nach weitgehender destillativer Abtrennung von gegebenenfalls unumgesetzten Chlorpropenen, von Chlorwasserstoff und überschüssigem o-Xylol, die dem Prozeß erneut zugeführt werden können, durch Behandlung mit Wasser bzw. Säuren unschädlich gemacht und entfernt.

Die weitere Reinigung des 2,2-Bis-(3,4-dimethy-phenyl)-propans kann, wie bereits beschrieben, durch Kristallisation erfolgen.

Die Prozentangaben der nachfolgenden Beispiele beziehen sich auf das Gewicht.

### Beispiele

### Vergleichsbeispiel

### (Molverhlältnis Dichlorpropan/Xylol = 1:4)

11,3 g (0,1 Mol) 2,2-Dichlorpropan, gelöst in 10,6 g (0,1 Mol) o-Xylol, werden innerhalb einer Stunde zu einer Lösung von 2,6 g AlCl₃ in 31,8 g (0,3 Mol) o-Xylol unter Rühren bei 0 bis 10°C (Eiskühlung) zugetropft Nach 4 Stunden Reaktionszeit bei dieser Temperatur wird durch Zugabe von 50 ml Wasser der Katalysator zerstört und entfernt. Nach Trennung der Phasen wird die wäßrige Phase noch zweimal mit o-Xylol (je 50 ml) extrahiert; die vereinigten organischen Phasen werden am Rotationsverdampfer im Vakuum eingeengt, wobei überschüssiges Xylol abdestilliert. Man erhält 19,1 g Rohprodukt, das nach GC-Analyse 67 % des gewünschten Produktes (= 51 % d.Th.) enthält. Das Rohprodukt wird mit 30 ml Methanol versetzt, die Mischung unter Rühren bis zum Rückfluß erhitzt und dann auf 0°C abgekühlt. Man erhält 11,4 g kristallines 2,2-Bis-(3,4-dimethyl-phenyl)-propan. Durch fraktionierte Destillation des Filtrates wird eine Fraktion (2,7 g) vom Siedebereich 120 bis 150°C/0,1 mbar erhalten, aus der durch erneute Kristallisation 0,8 g 2,2-Bis-(3,4-dimethyl-phenyl)-propan erhalten werden. Dies entspricht einer Gesamtausbeute von 12,2g (≙48 % d.Th.), bezogen auf eingesetztes 2,2-Dichlorpropan.

### Beispiel 1

### (Molverhältnis Dichlorpropan/Xylol = 1:10)

11,3 g (0,1 Mol) 2,2-Dichlorpropan, gelöst in 21,2 g (0,2 Mol) o-Xylol, werden innerhalb von 10 min zu einer Mischung von 1,1 g AlCl₃ und 85,0 g (0,8 Mol) o-Xylol unter Rühren bei 0 bis +10°C (Eiskühlung) zugetropft. Nach einer Reaktionszeit von 90 min wird durch Zugabe von 50 ml Wasser der Katalysator zerstört und entfernt. Nach Trennung der Phasen wird die wäßrige Phase noch zweimal mit o-Xylol (je 50 ml) extrahiert; die vereinigten organischen Phasen werden destilliert, wobei zunächst ein Wasseranteil azeotrop abdestilliert und danach überschüssiges o-Xylol zurückgewonnen wird. Der Destillationsrückstand wird mit 35 ml Methanol versetzt, die Mischung unter Rühren bis zum Rückfluß erhitzt und dann auf 0°C abgekühlt. Man erhält 14,2 g kristallines 2,2-Bis-(3,4-dimethyl-phenyl)-propan, Fp. 55,5°C. Aus dem Filtrat werden nach destillativer Entfernung des Lösungsmittels unter Normaldruck und von geringen Nebenproduktanteilen im Vakuum und erneuter Kristallisation weitere 9,7 g 2,2-Bis-(3,4-dimethyl-phenyl)-propan erhalten. Dies entspricht einer Gesamtausbeute von 91 % d.Th., bezogen auf eingesetztes 2,2-Dichlorpropan.

### Beispiel 2

### (Molverhältnis 2-Chlorpropen/Xylol = 1:12.5)

In 7,65 g einer Mischung aus 2-Chlor-propen (80 %) und E- und Z-1-Chlor-propen (20 %) (≙0,08 Mol 2-Chlor-propen) wird nach Zugabe von 0,1 g FeCl₃ Chlorwasserstoff bis zur Sättigung eingeleitet; dabei wird die Temperatur durch Kühlung mit Trockeneis bei -78°C gehalten. Nach 14 Stunden wird das erhaltene Reaktionsgemisch zu einer Mischung von 106 g (1 Mol) o-Xylol und 1,1 g AlCl₃ unter Rühren hinzugegeben, wobei die Temperatur zwischen -10 und +10°C gehalten wird. Nach 2 Stunden wird die Reaktion durch Zugabe von 100 ml Wasser beendet.

Die Aufarbeitung und Reinigung des erwünschten Reaktionsproduktes erfolgt analog Beispiel 1. Man erhält 17,5 g (= 87 % d.Th., bezogen auf eingesetztes 2-Chlor-propen) 2,2-Bis-(3,4-dimethy-phenyl)-propan.

### Beispiel 3

### (Molverhältnis 2-Chlorpropen/Xylol = 1:12.5)

Zu einer Lösung von 7,65 g 2-Chlor-propen (80 %) und E- und Z-1-Chlor-propenen (20 %) (≙0,08 Mol 2-Chlor-propen) in 106 g (1 Mol) o-Xylol werden 200 mg FeCl₃ hinzugefügt. Die Lösung wird bei 0°C mit Chlorwasserstoff gesättigt. Nach 15 Stunden werden 1,1 g AlCl₃ hinzugegeben, wobei ein Temperaturanstieg von 0 bis +20°C innerhalb einer Stunde erlaubt wird. Nach dieser Zeit wird die Reaktion durch Zugabe von 100 ml Wasser beendet. Die Phasen werden getrennt, die wäßrige Phase wird zweimal mit je 50 ml o-Xylol extrahiert und wieder abgetrennt.

Die weitere Aufarbeitung und Reinigung erfolgt analog Beispiel 1. Man erhält 16,8 g (= 83 % d.Th.) reines 2,2-Bis-(3,4-dimethyl-phenyl)-propan, Fp. 55 bis 56°C.

### Beispiel 4

### (Molverhältnis Dichlorpropan/Xylol = 1:5)

11,3 g (0,1 Mol) 2,2-Dichlorpropan werden innerhalb von 10 Minuten zu einer Lösung von 0,6 g AlCl₃ in 53 g (0,5 Mol) o-Xylol unter Rühren bei 0 bis 10°C zugetropft Nach 5 Stunden werden 50 ml Wasser zur Reaktionsmischung gegeben und die Phasen getrennt. Die wäßrige Phase wird zweimal mit o-Xylol (je 50 ml) extrahiert. Die vereinigten organischen Phasen werden durch eine Vakuumdestillation vom überschüssigen Xylol befreit; es hinterbleibt ein Rohprodukt (20,7 g), das aus 25 ml Methanol umkristallisiert wird Man erhält 14,3 g 2,2-Bis-(3,4-dimethy-phenyl)-propan; die Mutterlauge wird zunächst destillativ aufgearbeitet. Durch Umkristallisieren der Fraktion vom Siedebereich 135 bis 142°C/0,8 mbar aus Methanol erhält man weitere 4,0g des erwünschten Produktes. Die Gesamtausbeute an 2,2-Bis-(3,4-dimethyl-phenyl)-propan beträgt 73 % d.Th., bezogen auf eingesetztes 2,2-Dichlorpropan.

### Beispiel 5

### (Molverhältnis Dichlorpropan/Xylol = 1:6)

Analog Beispiel 4 werden 11,3 g (0,1 Mol) 2,2-Dichlorpropan mit 63,6 g (0,6 Mol) o-Xylol umgesetzt, wobei 0,7 g AlCl₃ als Katalysator verwendet werden. Nach 4 Stunden Reaktionszeit wird wie in Beispiel 4 beschrieben aufgearbeitet. Man erhält 19,9 g 2,2-Bis-(3,4-dimethyl-phenyl)-propan (Fp. 55 bis 55,5°C). Das entspricht einer Ausbeute von 79 % d.Th., bezogen auf eingesetztes 2,2-Dichlorpropan.

### Beispiel 6

### (Molverhältnis Dichlorpropan/Xylol = 1:8)

Analog Beispiel 4 werden 11,3 g (0,1 Mol) 2,2-Dichlorporpan mit 84,8 g (0,8 Mol) o-Xylol umgesetzt, wobei 0,9 g AlCl₃ als Katalysator verwendet werden. Nach 2,5 Stunden Reaktionszeit wird wie in Beispiel 4 beschrieben aufgearbeitet. Man erhält 21,1 g 2,2-Bis-(3,4-dimethyl-phenyl)-propan. Das entspricht einer Ausbeute von 84 % d.Th., bezogen auf eingesetztes 2,2-Dichlorpropan.

### Beispiel 7

275 g eines Chlorpropengemisches, nach GC-Analyse 68,6 % E/Z-1-Chlorpropene und 27,8 % 2-Chlor-propen enthaltend, wurde unter Zusatz von 1,3 g wasserfreiem Eisen-(III)-chlorid in einem 1 l-Rührautoklaven bei 10 bar mit Chlorwasserstoffgas behandelt. Die Reaktionstemperatur betrug 50°C, die Reaktionszeit 3 Stunden. Nach der Umsetzung enthielt das Reaktionsgemisch nach GC-Analyse 58,0 % E/Z-1-Chlorpropene und 39,3 % 2,2-Dichlorpropan, d.h die HCl-Addition erfolgte selektiv an das 2-Chlor-propen. Nach Behandlung des Rohgemisches mit Wasser und Trocknung der organischen Phase mit Calciumchlorid wurde das Gemisch durch fraktioniereride Destillation an einer 60 cm-Füllkörperkolonne getrennt. Man erhielt 99 g (= 88 % d.Th.) 2,2-Dichlorpropan vom Siedebereich 68-70°C.

## Patentansprüche

1. Verfahren zur Herstellung von 2,2-Bis-(3,4-dimethyl-phenyl)-propan durch Umsetzung von o-Xylol mit 2,2-Dichlorpropan in Gegenwart von Friedel-Crafts-Katalysatoren, wobei das Molverhältnis von eingesetztem o-Xylol zu eingesetztem 2,2-Dichlorpropan mindestens 5:1 beträgt.

2. Verfahren nach Anspruch 1, wobei das Molverhältnis mindestens 8:1 beträgt.

3. Verfahren nach Anspruch 1, wobei das Molverhältnis mindestens 10:1 betragt.

4. Verfahren nach Anspruch 1, dadurch gekennzeichnet, daß 2,2-Dichlorpropan verwendet wird, das aus 2-Chlor-propen durch Addition von Chlorwasserstoff hergestellt wurde.

5. Verfahren nach Anspruch 1, dadurch gekennzeichnet, daß 2,2-Dichlorpropan verwendet wird, das durch Umsetzung von 2-Chlor-propen mit Chlorwasserstoff in Gegenwart von 1-Chlor-propenen hergestellt wurde.

6. Verfahren nach Anspruch 5, dadurch gekennzeichnet, daß die Umsetzung mit o-Xylol ohne Abtrennung der 1-Chlorpropene oder aus ihnen entstandener Folgeprodukte ausgeführt wird.

## Claims

1. Process for the preparation of 2,2-bis-(3,4-dimethyl-phenyl)-propane by reaction of o-xylene with 2,2-dichloropropane in the presence of Friedel-Crafts catalysts, the molar ratio of the o-xylene employed to the 2,2-dichloropropane employed being at least 5:1.

2. Process according to Claim 1, the molar ratio being at least 8:1.

3. Process according to Claim 1, the molar ratio being at least 10:1.

4. Process according to Claim 1, characterised in that 2,2-dichloropropane which has been prepared from 2-chloro-propene by addition of hydrogen chloride is used.

5. Process according to Claim 1, characterised in that 2,2-dichloropropane which has been prepared by reaction of 2-chloro-propene with hydrogen chloride in the presence of 1-chloro-propenes is used.

6. Process according to Claim 5, characterised in that the reaction with o-xylene is carried out without the 1-chloropropenes or the secondary products formed from them being separated off.

## Revendications

1. Procédé de préparation de 2,2-bis-(3,4-diméthylphényl)propane par réaction de o-xylène avec du 2,2-dichloropropane en présence de catalyseurs Friedel-Crafts, où la proportion pondérale du o-xylène utilisé au 2,2-dichloropropane utilisée est au moins de 5 : 1.

2. Procédé selon la revendication 1, où la proportion molaire représente au moins 8 : 1.

3. Procédé selon la revendication 1, où la proportion molaire représente au moins 10 : 1.

4. Procédé selon la revendication 1, caractérisé en ce qu'on utilise du 2,2-dichloropropane, qu'on prépare à partir de 2-chloropropène par addition d'acide chlorhydrique.

5. Procédé selon la revendication 1, caractérisé en ce qu'on utilise du 2,2-dichloropropane qu'on prépare par réaction de 2-chloropropène avec du gaz chlorhydrique en présence de 1-chloropropènes.

6. Procédé selon la revendication 5, caractérisé en ce qu'on réalise la réaction avec du o-xylène sans fractionner les 1-chloropropènes ou les produits qui suivent et qui sont formés à partir d'eux.
